# EUROPEAN PATENT APPLICATION

(11) **EP 0 535 269 A1**
(43) Date of publication of application: **07.04.1993**
(21) Application number: 91116949.8
(22) Date of filing: 04.10.1991
(51) Int. Cl.: A61K 31/425, A61K 9/18

(54) **Pharmaceutical compositions containing thiazolidinedione derivatives**

(71) Applicant: TANABE SEIYAKU CO., LTD., Chuo-ku Osaka (JP)
(72) Inventor: Noda, Kazuo, Takarazuka-shi, Hyogo-ken (JP); Mizobe, Masakazu, Takatuki-shi, Osaka-fu (JP); Nakajima, Kingo, Katano-shi, Osaka-fu (JP); Yoneyama, Takashi, Suita-shi, Osaka-fu (JP)
(74) Representative: Hansen, Bernd, Dr.rer.nat.

(57) **Abstract**

A pharmaceutical preparation effective on diabetes on oral administration, which comprises, as an active ingredient, a benzoxazole derivative of the formula (I): 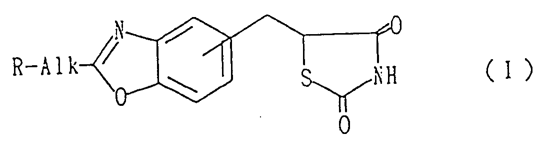
wherein R is optionally substituted phenyl or naphthyl group; and Alk is lower alkylene group or a pharmaceutically acceptable salt thereof characterized in that said active ingredient is dispersed or dissolved in a water-soluble polymer, and a method for the production thereof is provided.

## Description

The present invention relates to a pharmaceutical preparation having an improved in vivo absorption property. More specifically, it relates to an novel and effective oral antidiabetic in the form of a dispersed preparation in solid state or a solution.

As the number of patients suffering from diabetes increases, it became more and more important to develop novel and effective antidiabetics, and many kinds of drugs have been developed and provided. Although drugs may be in any form for administration, orally effective antidiabetic has advantages over other types of drugs because it can make the treatment easier and relieve patients of hardness possibly associated to injection or the like. This is important because diabetes generally takes a chronic course.

Accordingly, there has been an increased demand for the development of a novel antidiabetic drug which can be absorbed from digestive tract and is effective on oral administration.

It is known that a benzoxazole derivative of the formula (I): 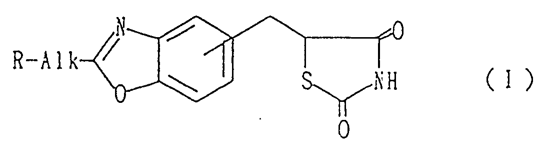
wherein R is optionally substituted phenyl or naphthyl group; and Alk is lower alkylene group or a pharmaceutically acceptable salt thereof is effective for the treatment of diabetes [European Patent Publication No. 0283035].

The above derivative has been demonstrated to have ability of stimulating and amplifying the sensitivity of a subject to insulin and to be useful as an antidiabetic drug. However, it shows a relatively low absorption rate from digestive tract, resulting in a poor bioavailability on oral administration.

The present inventors have studied intensively for the purpose of improving the absorption property of the benzoxazole compound (I) thereby providing a pharmaceutical preparation having a high and stable bioavailability on oral administration. We now have found that the absorption property of said compound can be surprisingly improved when it is formulated into a dispersed preparation in solid or liquid state. Such preparations can be formed by dispersing or dissolving the compound (I), as an active ingredient, into a dispersion medium, for example, a water-soluble polymer which is in solid or liquid state at room temperature. Since the above benzoxazole compound (I) and salts thereof are equivalent in the antidiabetic activity, when the term " benzoxazole derivative" is used to describe an active ingredient of the preparation of the invention, it also includes the salts thereof unless otherwise noted.

Thus, in one aspect, this invention provides a pharmaceutical preparation which comprises, as an active ingredient, a benzoxazole derivative of the formula (I): 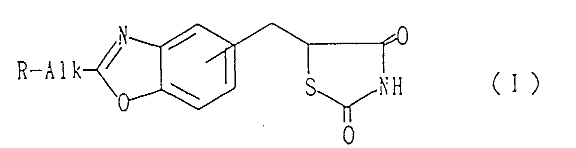
wherein R is optionally substituted phenyl or naphthyl group; and Alk is lower alkylene group or a pharmaceutically acceptable salt thereof characterized in that said active ingredient is dispersed or dissolved into a water-soluble polymer which is in solid or liquid state at room temperature.

The term "a solid dispersed powder or a solid dispersed preparation" refers to a pharmaceutical preparation comprising an active ingredient dispersed into a water-soluble polymer in solid state as a dispersion medium. Such a solid dispersed preparation comprises a benzoxazole derivative, as amorphous or fine powder, dispersed uniformly into a continuous solid phase of a water-soluble polymer which is solid at room temperature.

The term "a solution" ,when it is used to express a preparation of the invention, it refers to a pharmaceutical preparation comprising a benzoxazole derivative dissolved into a water-soluble polymer which is in liquid state at room temperature. In the specification, the term "a dispersed preparation in a water-soluble polymer in liquid state" is also used to express the "solution preparation" of the invention.

The term "lower alkyl" refers to a straight or branched saturated hydrocarbon radical having one to four carbon atoms, including methyl, ethyl, n-propyl, isopropyl, butyl, isobutyl, sec-butyl, tert-butyl and the like.

The term "lower alkylene" refers to alkylene groups having one to four carbon atoms, including methylene, ethylene, propylene, butylene and the like.

The term "(lower alkyl)amino" refers to a amino group substituted with lower alkyl. Examples of (lower alkyl)amino groups are dimethylamino, diethylamino and the like.

The term "halogen" refers to fluorine, chlorine, bromine, iodine and the like.

Although all the compound (I) as defined above can be used as an active ingredient of the present dispersed preparation, there are some preferable compounds. Preferred benzoxazole compounds are those wherein R is phenyl or naphthyl substituted with 1 - 3 substituents selected from a group consisting of halogen, di(lower alkyl)amino or pyrrolidino or a naphthyl group; and Alk is methylene. More preferably, R is a phenyl group substituted with halogen or di(lower alkyl)amino or pyrrolidino or a naphthyl group and Alk is methylene. Especially preferred compounds are 5-[(2,4-dioxothiazolidin-5-yl)methyl]-2-[4-(dimethylamino)benzyl]benzoxazole, 5-[(2,4-dioxothiazolidin-5-yl)methyl]-2-[(2-naphthyl)methyl]benzoxazole, 5-[(2,4-dioxothiazolidin-5-yl)methyl]-2-(4-chlorobenzyl)benzoxazole and 5-[(2,4-dioxothiazolidin-5-yl)methyl]-2-(4-pyrrolidinobenzyl)benzoxazole.

Examples of pharmaceutically acceptable salts are alkaline metal salts such as sodium salt, potassium salt and the like; alkaline earth metal salts such as calcium salt, magnesium salt and the like; and acid addition salts formed with mineral or organic acids such as hydrochloric acid, sulfuric acid and the like.

Water-soluble polymers which can be used in the present preparations are selected from those physiologically and pharmaceutically acceptable polymers. Examples of such polymers include polyethylene glycol (PEG), polyvinylpyrrolidone (PVP), hydroxypropyl cellulose (HPC), hydroxypropylmethyl cellulose (HPMC), methyl cellulose, gelatin, dextrin, carboxymethyl cellulose and the like, and salts thereof. Preferable water-soluble polymers are polyethylene glycol, polyvinylpyrrolidone, hydroxypropyl cellulose, hydroxypropylmethyl cellulose, methyl cellulose and dextrin. Especially preferred polymers for the present preparation are polyethylene glycol, polyvinylpyrrolidone, hydroxypropyl cellulose and hydroxypropylmethyl cellulose. Polyethylene glycol is in liquid or solid state depending on the molecular weight at room temperature. Preferable solid PEGs are those having an average molecular weight of about 1,000 to 20,000 and preferable liquid PEGs are those having an average molecular weight of about 200 to 600.

When the benzoxazole derivative of formula (I) is used in the form of a solid dispersed preparation, the preparation generally contains about 1 part by weight of the active compound (I) together with more than 1 part, preferably about 3 to about 50 parts, more preferably about 3 to about 20 parts by weight, of a water-soluble polymer which forms an continuous solid phase at room temperature. When the derivative of formula (I) is used in the form of a solution, the preparation generally contains about 1 part by weight of the active compound (I) together with about 3 to about 50 parts, more preferably about 30 to about 40 parts by weight, of a water-soluble polymer which is in liquid state at room temperature.

The solid dispersed preparation of the invention can be prepared by dispersing an active ingredient into a solid dispersion medium using conventional methods such as fusion or dissolution method. More specifically, it can be prepared by a fusion method which comprises mixing an active ingredient with a water-soluble polymer, which is in a solid state at room temperature, and heating the resultant mixture to obtain a melt which, upon cooling, gives a uniform solid dispersion. The fusion method can be carried out by mixing an active ingredient with a selected polymer of an appropriate size, and heating the mixture until it reaches to a molten state. Although heating temperature may vary depending on the nature of the polymer used, it can be carried out at a temperature ranging from about 60 to about 120°C, preferably from about 70 to about 100°C. The resultant molten mixture is cooled to a temperature where the polymer solidify.

Alternatively, the solid dispersed preparation of the invention can be obtained by a dissolution method which comprises dissolving an active ingredient and a water-soluble polymer in an appropriate solvent and removing the solvent from the resultant solution. The dissolution method can be carried out just by adding an active compound and a polymer to an appropriate solvent with stirring at room temperature or with heating to dissolve the both components, and removing the solvent from the solution. Although any solvents can be used so long as the both components dissolve therein, there are some preferable solvents such as organic solvents, for example, dimethylformamide, tetrahydrofuran, ethyl alcohol, methyl alcohol, chloroform and the like. Removal of solvent can be conducted conventionally, for example, by evaporating the solvent with heating or under reduced pressure with or without heating.

When the present preparation is in the form of solution, it can be prepared by dissolving an active compound into a liquid water-soluble polymer with heating, if necessary.

Thus, the present invention also provides a method for preparing an antidiabetic preparation clinically effective on oral administration, which comprises by dispersing or dissolving a benzoxazole derivative of the above formula (I) or a pharmaceutically acceptable salt thereof into a solid or liquid water-soluble polymer, respectively.

Thus obtained dispersed preparation or a solution can be administered without any further treatment. However, it can be formulated into a favorable formulations such as granules, fine granules, powders, soft capsules, hard capsules, tablets and the like together with known pharmaceutical additives such as excipient, binder, disintegrant, lubricant or the like by means of, for example, wet, dry or spray granulation method.

Examples of excipients employable for preparing the formulation of the invention include sugar, lactose, starch, sorbitol, mannitol, crystalline cellulose, calcium hydrogenphosphate, calcium citrate, calcium sulfate, hydroxypropyl cellulose and the like. Examples of binders include polyvinylpyrrolidone, cellulose derivatives such as methyl cellulose, ethyl cellulose, hydroxypropyl cellulose, hydroxypropylmethyl cellulose and the like, gelatin, starch paste, gum arabic, sodium alginate, sugar, polyvinyl alcohol, polyethylene glycol, methyl acrylate/metaacrylic acid copolymer and the like. When the binder is liquid and serves as solvent, it may be methanol, ethanol, water or the like. Examples of disintegrants include starch, kaolin, cellulose, sodium alginate, low-substituted hydroxypropyl cellulose, carboxymethyl cellulose, calcium carboxymethyl cellulose, crystalline cellulose, inner-bridged sodium carboxymethyl cellulose and the like. Examples of lubricants include alkaline metal stearate, alkaline metal lauryl sulfate, talc, precipitated calcium carbonate, synthetic aluminium silicate, silicon dioxide, magnesium oxide and the like.

When the formulation of the present invention is a solution, it may contain additives such as stabilizers, flavors, colorants and the like. Additives which can be used in a solution are selected from those generally used in the field of pharmaceutical industry, for example, methyl paraoxybenzoate, ethyl paraoxybenzoate, propyl paraoxybenzoate, butyl paraoxybenzoate, dehydroacetic acid, sodium dehydroacetate, sorbic acid, potassium sorbate, sodium sorbate, sodium glycyrrhizinate, disodium glycyrrhizinate, trisodium glycyrrhizinate, peppermint oil, fennel oil, β-carotin, l-menthol, tar pigments, lake pigments, caramel, iron oxides, cupric chlorophyll and the like.

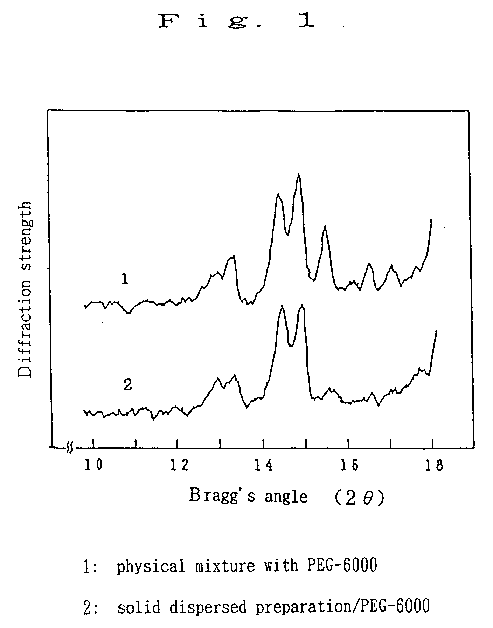

X-ray diffraction patterns of the solid dispersed preparation of the invention indicated that the crystalline benzoxazole compound (I) was converted into amorphous by the procedure of the invention (Fig.1). Furthermore, when a solid dispersed preparation or a solution of the invention containing a compound (I) and a water-soluble polymer was administered to dogs, the plasma levels of the active compound (I) in such dogs significantly increased, in comparison with the plasma levels in dogs treated with original compound (I) (raw material) or a physical mixture of said compound and water-soluble polymer. By virtue of the pharmaceutical preparation of the invention, an active compound (I) can be absorbed through digestive tract and express its pharmaceutical activity on oral administration. Thus, the present invention has successfully improved the bioavailability of benzoxazole derivative (I), thereby providing clinically effective oral antidiabetics.

As one of skill in the art will easily appreciate, the present method for improving the bioavailability of a drug is not limited to the use of benzoxazole derivatives (I), but is also applicable to any other drugs having a poor absorption property from digestive tract.

In the following drawings:
Fig. 1 is a comparison of x-ray diffraction patterns of a physical mixture (1) and a solid dispersed powder of the invention (2) each containing an active compound (a benzoxazole derivative (I)) and PEG-6000. Although, a background effect due to the crystalline property of the medium exists, it can be seen from the figure that a peak around the diffraction angle of 15.3° attributable to the crystalline compound (I) disappeared in the dispersed preparation, indicating that the crystalline compound (I) has been converted into amorphous.

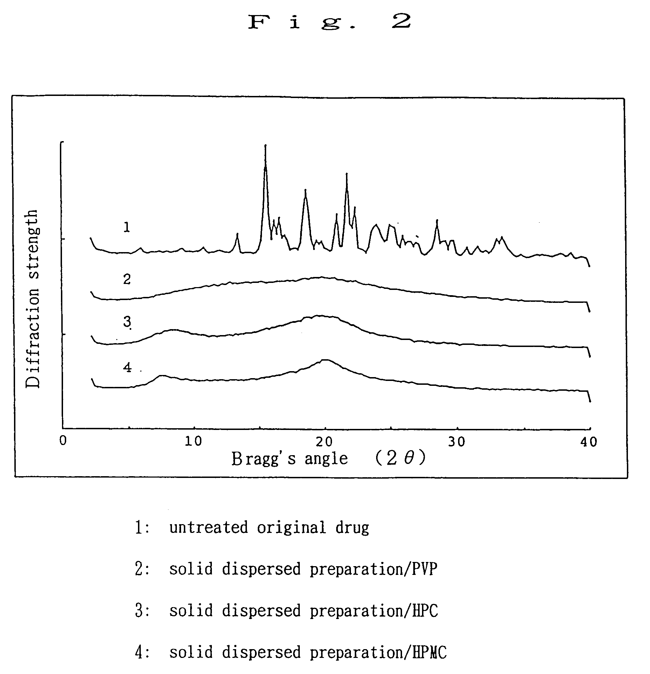

Fig. 2 is a comparison of powder X-ray diffraction patterns of various solid dispersed preparations of the invention each containing the same active compound (I) and a different dispersion medium therefor. In the figure, 1 represents untreated original drug (raw material), and 2, 3 and 4 each represents a solid dispersed powder formed by the use of PVP, HPC and HPMC as dispersion medium, respectively. It can be seen from the figure that the peak disappeared in all the dispersed preparations, demonstrating the usefulness of these three media.

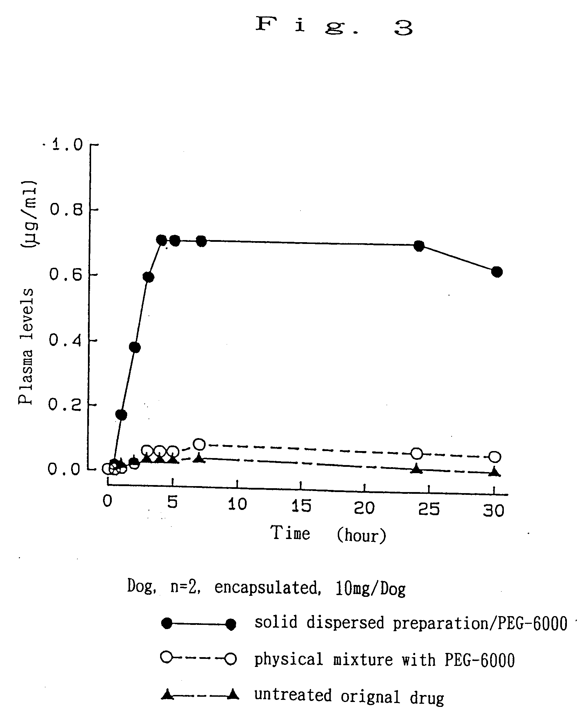

Fig.3 shows the time-course of the plasma levels of an active compound (I) following the oral administration of a solid dispersed preparation of the invention in dogs. In the figure, closed circles represent the plasma levels of the compound (I) after the administration of a solid dispersed powder containing said compound (I) and PEG-6000, open circles represent the plasma levels after the administration of a physical mixture thereof, and closed triangles represent the plasma levels after the administration of untreated original drug. The figure indicates that the solid dispersed preparation of the invention increased the plasma level of compound (I) significantly, compared with the physical mixture and the original drug.

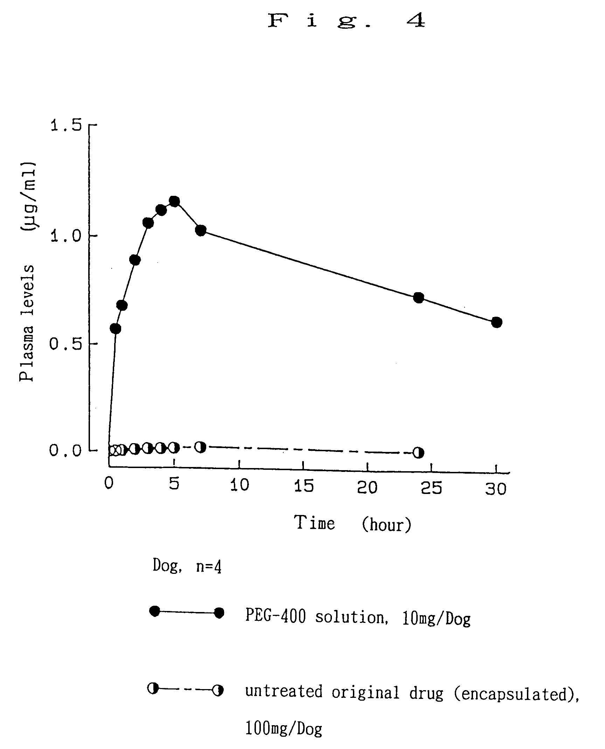

Fig.4 shows the time-course of the plasma levels of an active compound (I) following the oral administration of a solution of said compound in a water-soluble polymer, PEG-400, in dogs. In the figure, closed circles represent the plasma levels after the administration of polymer solution and semi-closed circles represent the plasma levels after the administration of untreated original drug. The results show that the plasma level of compound (I) is significantly high when the solution of the invention was administered, whereas it is extremely low when the raw material was administered.

The following Examples further illustrate the present invention. The Examples are not intended to be limiting to the scope of the invention in any respect and should not be so construed.

The following experiments were conducted to demonstrate that the crystalline benzoxazole derivative (I) was successfully converted into amorphous powder by the process of the invention and that the bioavailability of said compound on oral administration have been increased in the dispersed preparation of the invention.

### Experiment 1

### X-Ray Diffraction Analysis of a Solid Dispersed Powder Containing PEG-6000 as Dispersion Medium

### (1) Preparation of a Solid Dispersed Powder

Ten gram of 5-[(2,4-dioxothiazolidin-5-yl)methyl]-2-[(2-naphthyl)methyl]benzoxazole (average particle size : about 20 µm) was combined with 323 g of PEG-6000 (polyethylene glycol 6000) and the mixture (content of active ingredient : 3%) was mixed intimately, heated at 80°C for 30 minutes to obtain a solution. The resultant polymer solution was allowed to stand for at room temperature to solidify the polymer. The solid was ground by means of a mortar and passed through a No.32 mesh sieve to obtain a solid dispersed powder.

### (2) X-Ray Diffraction Analysis

The solid dispersed powder prepared in the above (1) and a physical mixture (control) prepared by blending the same components at the same ratio were subjected to the X-ray diffractometory. The results are given in the accompanying Fig. 1. Although the determination of the exact crystallization rate was rather difficult due to the background effect caused by the medium which gives crystalline-like diffraction spectra, it can be seen from the X-ray diffraction patterns of Fig. 1 that a peak around 15.3° of diffraction angle, which is attributable to the crystalline benzoxazole compound (I), disappeared in the solid dispersed preparation. This means the crystalline benzoxazole (I) had been converted into amorphous powder in the solid dispersed preparation.

### Experiment 2

### X-Ray Diffraction Analysis of Various Solid Dispersed Powders

### (1) Preparation of Solid Dispersed Powders

One gram of 5-[(2,4-dioxothiazolidin-5-yl)methyl]-2-[(2-naphthyl)methyl]benzoxazole (average particle size : about 20 µm) was combined with 4 g of PVP (polyvinylpyrrolidone) at the mixing ratio of 1:4 by weight of an active ingredient to a polymer. The mixture was dissolved into 35 ml of dimethylformamide and the solution was evaporated to dryness under reduced pressure to yield a solid residue. The residue was then ground by means of a mortar and passed through a No.32 mesh sieve to obtain a solid dispersed powder. The same procedure was repeated just by replacing PVP with HPC (hydroxypropyl cellulose) or HPMC (hydroxypropylmethyl cellulose) to obtain two more preparations.

### (2) X-Ray Diffraction Analysis

The solid dispersed powders prepared in the above (1) were subjected to the X-ray diffractometory. The results are shown in the accompanying Fig. 2, which gives a comparison of powder X-ray diffraction patterns of the above three solid dispersed preparations. In the figure, 1 represents the control, i.e.,untreated original drug, and 2, 3 and 4 each represents a solid dispersed powder formed by the use of PVP, HPC and HPMC, respectively. It can be seen from the figure that the peak around the diffraction angle of 15.3° completely disappeared in all the dispersed preparations, indicating that the active compound (I) had been converted into amorphous in these preparations.

### Experiment 3

### Bioavailability of Solid Dispersed Preparation

The in vivo absorption rate from digestive tract of a solid dispersed powder was determined in dogs.

Two dogs (12 - 13 kg) were used in this experiment. A solid dispersed powder containing 5-[(2,4-dioxothiazolidin-5-yl)methyl]-2-[(2-naphthyl)methyl]benzoxazole and PEG-6000 was prepared according to the procedure described in Experiment 1. The preparation was encapsulated (0.333 g /capsule) and administered orally (10 mg of active ingredient/Dog). In the separate experiment, a physical mixture containing said compound and the medium at the same concentration was prepared by blending them, and administered to dogs at the same administration rate (10 mg/Dog). The untreated original drug was also administered (10 mg/Dog).

Blood samples were taken from the dogs until 30 hours postadministration. The concentration of the active compound (I) in the plasma sample was assayed by HPLC method. The results are shown in Fig. 3.

In the figure, closed circles represent the average plasma levels of the compound (I) following the administration of the solid dispersed powder, open circles represent those following the administration of a physical mixture, and closed triangles represent those following the administration of untreated original drug. As apparent from the figure, the plasma levels of the drug after the administration of the solid dispersed preparation is significantly high compared with those after the administration of the untreated original drug or the physical mixture. This result shows that the bioavailability of an active compound (I) have been improved significantly in the dispersed preparations.

### Experiment 4

### Bioavailability of a Solution in a Water-soluble Polymer

### (1) Preparation of a Solution in a Water-soluble Polymer

5-[(2,4-dioxothiazolidin-5-yl)methyl]-2-[(2-naphthyl)methyl]benzoxazole (3 g) was dissolved in polyethyleneglycol (average molecule weight: approximately 380-420, trade name: Macrogol-400, Sanyo Chemical Industries Ltd.) (100 ml) to provide the preparation of the present invention. Mixture of an equal amount of untreated original drug (particle size: 20 µm) and corn starch was encapsulated in a gelatin capsule to give a formulation used as a control.

### (2) Assay of Bioavailability

Four dogs (10 - 15 kg) were used in this experiment. The preparation of the invention was administered to dogs orally at the dose of 10 mg/Dog. In the separate experiment, the control formulation was administered at 100 mg/Dog. The blood was taken from the dogs and the concentration of the active compound (I) in the plasma sample was assayed in the same manner as described in the above. The time-course of the plasma levels of the drug was plotted. The results are shown in Fig. 4.

In the figure, closed circles represent the average plasma levels of the compound (I) following the administration of the solution of the invention, and semi-closed circles represent those following the administration of untreated original drug. As apparent from the figure, the plasma levels of the drug after the administration of the solution is significantly high compared with those after the administration of the control formulation.

### Example 1

### (1) Preparation of Solid Dispersed Powder

5-[2,4-dioxothiazolidin-5-yl)methyl]-2-[2-(naphthyl)methyl]benzoxazole (10 g) was mixed with polyethyleneglycol (average molecular weight: approximately 7300-9300, trade name: Macrogol-6000, Sanyo Chemical Industries Ltd.) (90 g), and the mixture was heated at 120°C for 30 minutes until the mixture melts. Then, the resulting melt was kept at room temperature to solidify, pulverized in a mortar, passed through a No. 32 mesh sieve to provide solid dispersed powder.

### (2) Preparation of Tablets

The product from the above (1) (100 parts by weight) was mixed with corn starch (73.3 parts by weight). The mixture was admixed with aqueous ethanol solution containing polyvinylpyrrolidone (5.4 parts by weight). The admixture was passed through a No. 24 mesh JIS standard sieve, and the substances passed through the sieve were dried at 45°C for 3 hours. After drying the mixture, magnesium stearate (0.9 parts by weight) was added thereto and mixed. The mixture was compressed on a rotary tablet machine to yield tablets each weighing 180 mg and having a diameter of 8 mm.

### Example 2

### (1) Preparation of Solid Dispersed Powder

The same procedure as described in the above Example 1, (1) was repeated to obtain solid dispersed powder except that 5-[(2,4-dioxothiazolidin-5-yl)methyl]-2-[(2-naphthyl)methyl]benzoxazole (3 g) was used as an active compound and the heating was carried out at 80°C for 30 minutes.

### (2) Preparation of Powder

The product obtained in the above (1) (93 parts by weight) and corn starch (81 parts by weight) was mixed, and admixed with aqueous ethanol solution containing hydroxypropyl cellulose (6 parts by weight). The admixture was dried at 45°C for 15 minutes and pulverized. Then the resultant product was dried at 45°C for 3 hours and passed through No. 32 mesh JIS standard sieve to obtain powder.

### Example 3

### (1) Preparation of Dispersed Powder

5-[(2,4-dioxothiazolidin-5-yl)methyl]-2-(4-chlorobenzyl)benzoxazole (3 g) and polyvinylpyrrolidone (average molecular weight: approximately 49000, trade name: Kollidon30, BASF Co. Ltd.) (15 g) were dissolved into dimethylformamide (12 ml). Then, the solvent was evaporated in vacuo, and the residue was pulverized in a mortar and passed through a No. 24 mesh JIS standard sieve to obtain solid dispersed powder.

### (2) Preparation of Tablet

The product obtained in the above (1) (90 parts by weight), crystalline cellulose (10 parts by weight) and low-substituted hydroxypropyl cellulose (9 parts by weight) were mixed together. To the mixture was added magnesium stearate (1 part by weight) and mixed. Tablets each weighing 110 mg and having 7 mm of diameter were obtained by repeating the same procedure as above Example 1, (2).

### Example 4

### (1) Preparation of Solid Dispersed Powder

The same procedure as described in the above Example 3, (1) was repeated employing 5-[(2,4-dioxothiazolidin-5-yl)methyl]-2-[(2-naphthyl)methyl]benzoxazole (5 g) and hydroxypropylmethyl cellulose (trade name: TC-5, Shin-etsu Chemical Co. Ltd.) (15 g) to obtain solid dispersed powder.

### (2) Preparation of Tablet

The product (150 parts by weight) obtained in the above (1), lactose (230 parts by weight) and calcium carboxymethyl cellulose (50 parts by weight) were mixed. The mixture was granulated with aqueous ethanol solution containing hydroxypropyl cellulose (15.5 parts by weight) being sprayed thereto. Then, the resulting particles were dried at 45°C for 30 minutes, pulverized, passed through No. 24 mesh JIS standard sieve, mixed with magnesium stearate (4.5 parts by weight) and worked up in the same way as Example 1, (2) to give tablets each weighing 180 mg.

### Example 5

### Preparation of Capsule

Solid dispersed powder obtained in the above Example 2 was filled into hard gelatin capsules to give capsule formulation.

### Example 6

(1) The same procedure as described in Example 1, (1) was repeated employing 5-[(2,4-dioxothiazolidin-5-yl)methyl]-2-[(2-naphthyl)methyl]benzoxazole (10 g) and polyethyleneglycol (average of molecular weight; approximately 7300-9300, trade name: Macrogol-6000, Sanyo Chemical Industries Ltd.) (90 g) to obtain solid dispersed powder.

### (2) Preparation of Granule

The above product (100 parts by weight) and corn starch (90 parts by weight) were mixed. To the mixture was added aqueous ethanol solution containing hydroxypropyl cellulose (10 parts by weight) and admixed. The admixture was dried at 45°C for 15 minutes and pulverized. Then, the substance was passed through a No. 12 mesh JIS standard sieve to obtain granules.

### Example 7

### (1) Preparation of Solid Dispersed Powder

The same procedure as described in Example 4, (1) was repeated employing 5-[(2,4-dioxothiazolidin-5-yl)methyl]-2-[(2-naphthyl)methyl]benzoxazole (5 g) and hydroxypropyl cellulose (trade name: HPC, Nippon Soda Co. Ltd.) (15 g) to provide solid dispersed powder.

### (2) Preparation of Powder

The product (100 parts by weight) obtained in the above (1) and corn starch (95 parts by weight) were mixed. To this mixture was added aqueous ethanol solution containing hydroxypropyl cellulose (5 parts by weight) and admixed. The admixture was dried at 45°C for 15 minutes and pulverized. Then, the resultant product was dried at 45°C for 3 hours and passed through a No. 32 mesh JIS standard sieve to provide powder formulation.

### Example 8

### Preparation of Solution

5-[(2,4-dioxothiazolidin-5-yl)methyl]-2-[(2-naphthyl)methyl)benzoxazole (3 g) was dissolved in polyethyleneglycol (average molecular weight: approximately 380-420, trade name: Macrogol-400, Sanyo Chemical Industries Ltd.) (100 ml) to obtain solution.

### Example 9

### (1) Preparation of Solid Dispersed Powder

The same procedure as described in Example 1, (1) was repeated to obtain solid dispersed powder except that 5-[(2,4-dioxothiazolidin-5-yl)methyl]-2-(4-pyrrolidinobenzyl)benzoxazole (2 g) was used as an active compound and the heating was carried out at 80°C for 30 minutes.

### (2) Preparation of Powder

The product obtained in the above (1) (62 parts by weight) and corn starch (54 parts by weight) was mixed, and admixed with aqueous ethanol solution containing hydroxypropyl cellulose (4 parts by weight). The admixture was dried at 45°C for 15 minutes and pulverized. Then the resultant product was dried at 45°C for 3 hours and passed through No. 32 mesh JIS standard sieve to obtain powder.

### Example 10

### (1) Preparation of Solid Dispersed Powder

The same procedure as described in Example 3, (1) was repeated employing 5-[(2,4-dioxothiazolidin-5-yl)methyl]-2-(4-pyrrolidinobenzyl)benzoxazole (2.5 g) and hydroxypropylmethyl cellulose (trade name: TC-5, Shin-etsu Chemical Co. Ltd.) (7.5 g) to obtain solid dispersed powder.

### (2) Preparation of Tablet

The product (92 parts by weight) obtained in the above (1), lactose (140 parts by weight) and calcium carboxymethyl cellulose (31 parts by weight) were mixed. The mixture was granulated with aqueous ethanol solution containing hydroxypropyl cellulose (9.5 parts by weight) being sprayed thereto. Then, the resulting particles were dried at 45°C for 30 minutes, pulverized, passed through No. 24 mesh JIS standard sieve, mixed with magnesium stearate (2.8 parts by weight) and worked up in the same way as Example 1, (2) to give tablets each weighing 110 mg.

## Claims

1. A pharmaceutical preparation which comprises, as an active ingredient, a benzoxazole derivative of the formula (I): 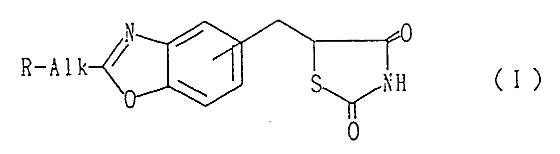 wherein R is optionally substituted phenyl or naphthyl group; and Alk is lower alkylene group or a pharmaceutically acceptable salt thereof characterized in that said active ingredient is dispersed or dissolved in a water-soluble polymer.

2. The pharmaceutical preparation of Claim 1, wherein an active ingredient is dispersed into a water-soluble polymer which is in solid state at room temperature or dissolved into a water-soluble polymer which is in liquid state at room temperature.

3. The preparation of Claim 1, wherein the water-soluble polymer is selected from the group consisting of polyethylene glycol, polyvinylpyrrolidone, hydroxypropylmethyl cellulose and hydroxypropyl cellulose.

4. The preparation of Claim 1, wherein the active ingredient is a benzoxazole derivative of formula (I) wherein R is a phenyl group or a naphthyl group optionally substituted with 1 to 3 substituents selected from the group consisting of halogen atoms, di(lower alkyl)amino groups and pyrrolidino group.

5. The preparation of Claim 1, wherein R is a phenyl group substituted with halogen atom, di(lower alkyl)amino group or pyrrolidino group, or a naphthyl group.

6. The preparation of Claim 1, wherein the active ingredient is 5-[(2,4-dioxothiazolidin-5-yl)methyl]-2-[4-(dimethylamino)-benzyl]benzoxazole or a pharmaceutically acceptable salt thereof.

7. The preparation of Claim 1, wherein the active ingredient is 5-[(2,4-dioxothiazolidin-5-yl)methyl]-2-[(2-naphthyl)methyl]benzoxazole or a pharmaceutically acceptable salt thereof.

8. The preparation of Claim 1, wherein the active ingredient is 5-[(2,4-dioxothiazolidin-5-yl)methyl]-2-(4-chlorobenzyl)benzoxazole or a pharmaceutically acceptable salt thereof.

9. The preparation of Claim 1, wherein the active ingredient is 5-[(2,4-dioxothiazolidin-5-yl)methyl]-2-(4-pyrrolidinobenzyl)benzoxazole or a pharmaceutically acceptable salt thereof.

10. A method for producing an antidiabetic preparation clinically effective on oral administration, which comprises dispersing a benzoxazole derivative of the formula (I) or a pharmaceutically acceptable salt thereof into a water-soluble polymer in solid state or dissolving into a water-soluble polymer in liquid state.

11. The method of Claim 10, wherein the dispersion process is conducted by heating a mixture of said compound (I) and a water-soluble polymer until the mixture melts, and cooling the molten mixture to allow the mixture solidify, or by dissolving said compound (I) and a water-soluble polymer into a solvent, and removing the solvent from the resultant solution.

12. A pharmaceutical formulation for treating a subject suffering from diabetes, which comprises a preparation of Claim 1 together with pharmaceutically acceptable additives therefor.

13. The use of a preparation according to any of Claims 1 to 9 for preparing an orally applicable pharmaceutical against diabetes.

## Amended claims

### Amended claims in accordance with Rule 86(2) EPC.

1. A method for preparing a pharmaceutical composition which comprises dispersing or dissolving a benzoxazole derivative of the formula (I): 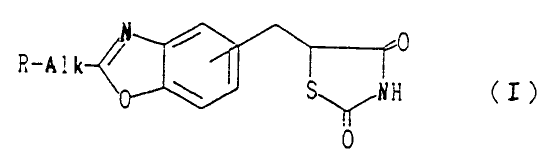 wherein R is optionally substituted phenyl or naphthyl group; and Alk is lower alkylene group or a pharmaceutically acceptable salt thereof as an active ingredient in a water-soluble polymer.

2. The method of claim 1 wherein the active ingredient is dispersed into a water-soluble polymer which is in solid state at room temperature or dissolved into a water-soluble polymer which is in liquid state at room temperature.

3. The method of claim 1 wherein the water-soluble polymer is selected from the group consisting of polyethylene glycol, polyvinylpyrrolidone, hydroxypropylmethyl cellulose and hydroxypropyl cellulose.

4. The method of claim 1 wherein the active ingredient is a benzoxazole derivative of formula (I) wherein R is a phenyl group or a naphthyl group optionally substituted with 1 to 3 substituents selected from the group consisting of halogen atoms, di(lower alkyl)amino groups and pyrrolidino groups.

5. The method of claim 1, wherein R is a phenyl group substituted with halogen atom, di(lower alkyl)amino group or pyrrolidino group, or a naphthyl group.

6. The method of claim 1, wherein the active ingredient is 5-[(2,4-dioxothiazolidin-5-yl)methyl]-2-[4-(dimethylamino)-benzyl]benzoxazole or a pharmaceutically acceptable salt thereof.

7. The method of claim 1, wherein the active ingredient is 5-[(2,4-dioxothiazolidin-5-yl)methyl]-2-[(2-naphthyl)methyl] benzoxasole or a pharmaceutically acceptable salt thereof.

8. The method of claim 1, wherein the active ingredient is 5-[(2,4-dioxothiazolidin-5-yl)methyl)-2-(4-chlorobenzyl)benzoxazole or a pharmaceutically acceptable salt thereof.

9. The method of claim 1, wherein the active ingredient is 5-[(2,4-dioxothiazolidin-5-yl)methyl]-2-(4-pyrrolidinobenzyl) benzoxazole or a pharmaceutically acceptable salt thereof.

10. A method for preparing a pharmaceutical composition according to any of claims 1 to 9 by formulating said composition with pharmaceutically acceptable additives therefor.

11. A method for producing an antidiabetic preparation clinically effective on oral administration, which comprises dispersing a benzoxazole derivative of the formula (I) 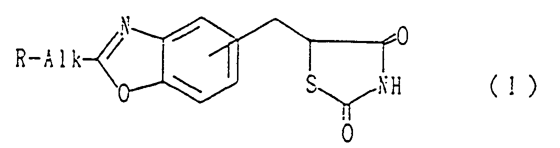 wherein R is optionally substituted phenyl or naphthyl group; and Alk is lower alkylene group or a pharmaceutically acceptable salt thereof into a water-soluble polymer in solid state or dissolving into a water-soluble polymer in liquid state.

12. The method of claim 11, wherein the dispersion process is conducted by heating a mixture of said compound (I) and a water-soluble polymer until the mixture melts, and cooling the molten mixture to allow the mixture solidify, or by dissolving said compound (I) and a water-soluble polymer into a solvent, and removing the solvent from the resultant solution.

13. The use of a pharmaceutical composition according to any of claims 1 to 9 for the preparation of an orally applicable pharmaceutical against diabetes.
